# EUROPEAN PATENT APPLICATION

(11) **EP 1 992 299 A2**
(43) Date of publication of application: **19.11.2008**
(21) Application number: 08005757.3
(22) Date of filing: 27.03.2008
(51) Int. Cl.: A61B 17/72

(54) **Apparatus for securing a bone screw to an intramedullary nail**

(30) Priority: 15.05.2007 US 748772
(71) Applicant: Zimmer Technology, Inc., Warsaw, IN 46580 (US)
(72) Inventor: Keith, Michael, Winona Lake Indiana 46590 (US); Cordray, Michael, Columbia City Indiana 46725 (US); Kitch, Steve, Akron Indiana 46910 (US); Lower, Jerry, Bourbon Indiana 46504 (US); Koser, Tony, Warsaw Indiana 46580 (US); Wack, Michael W., Warsaw Indiana 46580 (US)
(74) Representative: Mays, Julie

(57) **Abstract**

An intramedullary nail configured for use in a bone, the nail including at least one bore configured to receive a bone screw. The bore includes at least one member configured to engage the threads of the screw to prevent axial motion of the screw within the bore after application. The member may be integrally formed in the bore or may be a separate member added to the bore. The member may have a variety of cross sectional shapes, such as a rectangular shape, circular shape or a triangular shape.

## Description

### BACKGROUND

### 1. Field of the Invention.

The present invention relates generally to an intramedullary nail for use in assisting in the healing of bones.

### 2. Description of the Related Art.

The usage of intramedullary nails to assist in the healing of bones by locating the intramedullary nail in the canal of a bone is known. Bone screws are often employed to help retain the nail at a desired location within the bone canal. Often the bone screws are inserted into bores formed in the intramedullary nail. The bores generally have substantially smooth side walls thereby allowing the screw to pass freely through the nail.

### SUMMARY

An intramedullary nail configured for use in a bone, the nail including at least one bore configured to receive a bone screw. The bore includes at least one member configured to engage the threads of the screw to prevent axial motion of the screw within the bore after application. The member may be integrally formed in the bore or may be a separate member added to the bore. The member may have a variety of cross sectional shapes, such as a rectangular shape, circular shape or a triangular shape.

In one form, the present invention provides for an intramedullary nail configured to receive at least a portion of a bone screw, the nail including a body portion having first and second ends; and at least one bore in the body portion, the bore configured to receive the bone screw, wherein at least a portion of the bore is substantially smooth and the bore comprises at least one member configured to engage at least a portion of a thread of the screw.

In another form, the present invention provides for an intramedullary nail configured for use in a canal of a bone, the nail includes a first end including at least one bore configured to receive a screw, a second end, a body portion intermediate the first end and the second end wherein the at least one bore includes a substantially smooth portion and means for preventing movement of the screw with respect to the nail when the screw is not rotating.

In another form, the present invention provides for a method of forming a bore configured to engage a screw in an intramedullary nail having a distal end, a proximal end and a body positioned intermediate the distal end and the proximal end, the method including the steps of milling a bore sized to receive the screw in one of the proximal or distal ends, wherein a portion of the bore includes a substantially smooth portion, and fashioning a member in the bore configured to engage the screw as the screw is inserted into the bore.

### BRIEF DESCRIPTION OF THE DRAWINGS

The above-mentioned and other features and advantages of this invention, and the manner of attaining them, will become more apparent and the invention itself will be better understood by reference to the following description of embodiments of the invention taken in conjunction with the accompanying drawings, wherein:

Figure 1 is a perspective view of an intramedullary nail embodying an aspect of the present invention;

Figure 2 is an enlarged fragmentary view of a portion of the nail depicted in Figure 1;

Figure 3 is a side view of the portion of the nail depicted in Figure 2;

Figure 4 is a section view taken along section line 4-4 in Figure 2 depicting an embodiment of an engagement portion within a bore of the nail;

Figure 5 is a perspective view of a screw;

Figure 6 is a perspective view of the screw depicted in Figure 5 inserted into the nail depicted in Figure 1;

Figure 7 is a section view taken along the section line 7-7 of Figure 6;

Figure 8 is an enlarged section view of the area indicated in Figure 7;

Figures 9A-9C are section views depicting an exemplary method of forming a portion of the bores present within the nail depicted in Figure 1;

Figure 10 is a section view depicting an alternative embodiment of an engagement portion present within a bore of the nail;

Figure 11 is section view depicting an alternative embodiment of engagement portions present within a bore of the nail;

Figure 12 is a perspective view depicting an exemplary method of forming the engagement portions depicted in Figure 11;

Figure 13 is a side view of a bore including an alternative embodiment of an engagement portion formed in a bore of the nail depicted in Figure 1;

Figures 14 through 17 are section views depicting an alternative configuration of engagement portions present within a bore of the nail;

Figures 18A-18C are section views depicting an exemplary method of forming a portion of the bores present within a nail;

Figure 19 is a perspective view of an intramedullary nail embodying aspects of the present invention; and

Figure 20 is a perspective view of an elongated bore embodying aspects of the present invention.

Corresponding reference characters indicate corresponding parts throughout the several views. The exemplifications set out herein illustrate embodiments of the invention, and such exemplifications are not to be construed as limiting the scope of the invention in any manner.

### DETAILED DESCRIPTION

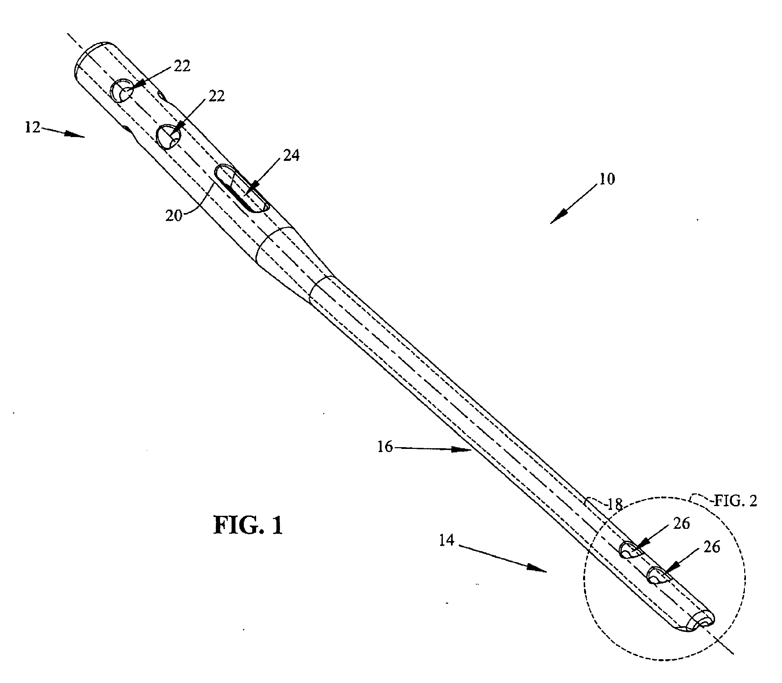

Figure 1 depicts an intramedullary nail, generally indicated by numeral 10, representing an embodiment of the presenting invention. Nail 10 includes a proximal end 12, a distal end 14 and a body portion 16 located intermediate the ends 12, 14. Nail 10 may be formed from any suitable biocompatible material, such as titanium or stainless steel. Nail 10 may also have any shape suitable for any surgical techniques. Nail 10 is not intended to be limited to use in any particular body part or bone, such as a femur.

Nail 10 further includes a cannula 18, shown in Figure 1 in phantom, extending substantially along the longitudinal axis 20 of nail 10. In the depicted embodiment, cannula 18 begins at proximal end 12 and terminates at distal end 14. In embodiments, cannula 18 may traverse only a portion of nail 10, if desired.

Referring still to Figure 1, proximal end 12 includes a plurality of through bores, each indicated by numeral 22. In the depicted embodiment, bores 22 extend completely through nail 10, and generally have circular shapes. Proximal end 12 also includes an elongated bore, generally indicated by numeral 24. Elongated bore 24 may have substantially the same structure as bores 22 but has an elongated shape.

Distal end 14 of nail 10 may be configured to be inserted into a canal of a bone in a known manner. In the depicted embodiment, distal end 14 includes a plurality of bores, each indicated by numeral 26. In the depicted embodiment, bores 26 each have a circular shape and extend entirely through nail 10.

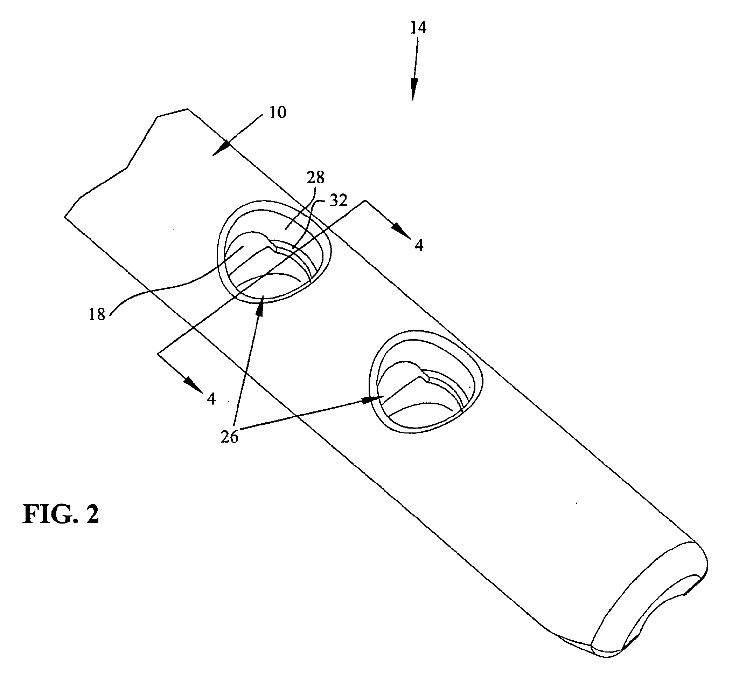

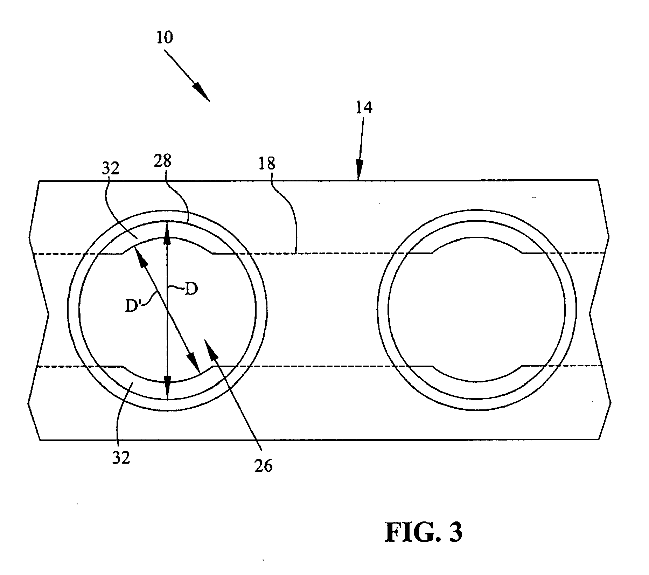

With reference to Figures 1-4, in the depicted embodiment, bore 26 includes a first smooth portion, generally indicated by numeral 28, a second smooth portion, generally indicated by numeral 30, and engagement portions, generally indicated by numeral 32. In the depicted embodiment, the smooth portions 28, 30 define substantially similar diameters, indicated by D in Figure 3, of bore 26. In the depicted embodiment, the substantially smooth portions 28, 30 form, or extending along, at least a majority of the length of bore 26. The substantially smooth portions 28, 30 may form, or extend along, at least 85%, 90% or 95% of the length of bore 26. In embodiments, the smooth portions 28, 30 may form a larger portion of the bore 26, if necessary.

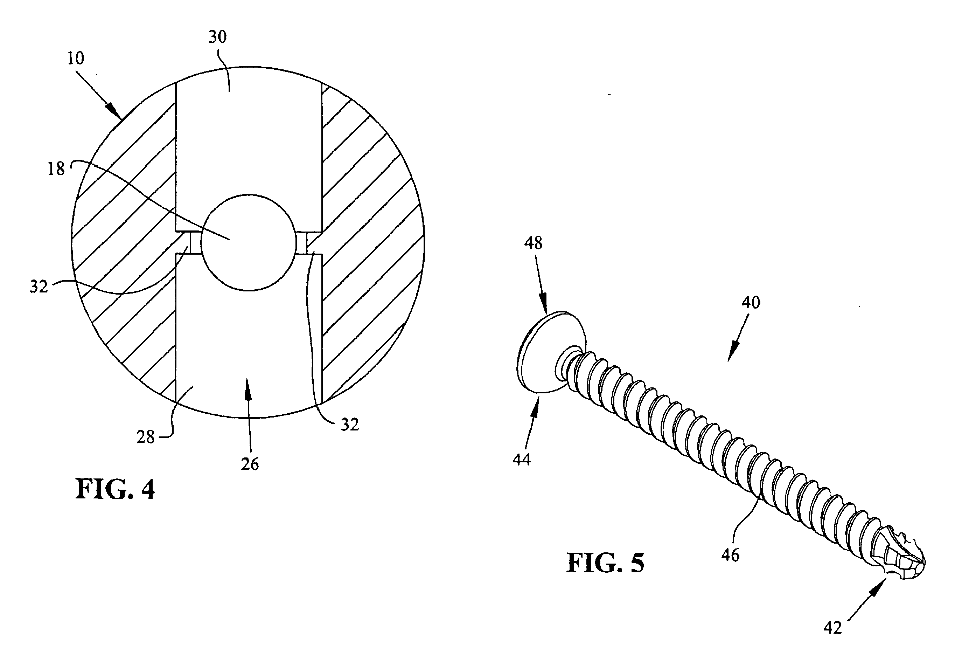

Engagement portions 32 also define a diameter, indicated by D', in bore 26. In the present embodiment, diameter D is greater in magnitude than diameter D'. As depicted in Figure 4, engagement portions 32 have a substantially rectangular cross section. In addition, as depicted in Figure 4, engagement portions 32 are integrally formed with nail 10. As shown in Figure 3, cannula 18 bisects the engagement portions 32, thereby forming the two engagement portions 32. It should be noted that if desired, the engagement portions 32 may be formed at a position spaced apart from cannula 18 thereby creating an annular engagement portion.

Figure 5 depicts a perspective view of a bone screw, indicated by numeral 40. Bone screw 40 may be of any type know in the art and may be configured for any surgical technique requiring screw 40 to mate with nail 10. In general, bone screw 40 includes a distal end 42, a proximal end 44 and a thread 46 extending from distal end 42 to proximal end 44. In the depicted embodiment, proximal end 44 includes a head portion 48 configured to provide a mechanism for driving screw 40. It should be noted that in embodiments of screw 40, thread 46 need not extend entirely from distal end 42 to proximal end 44, but may be interrupted with a substantially smooth portion.

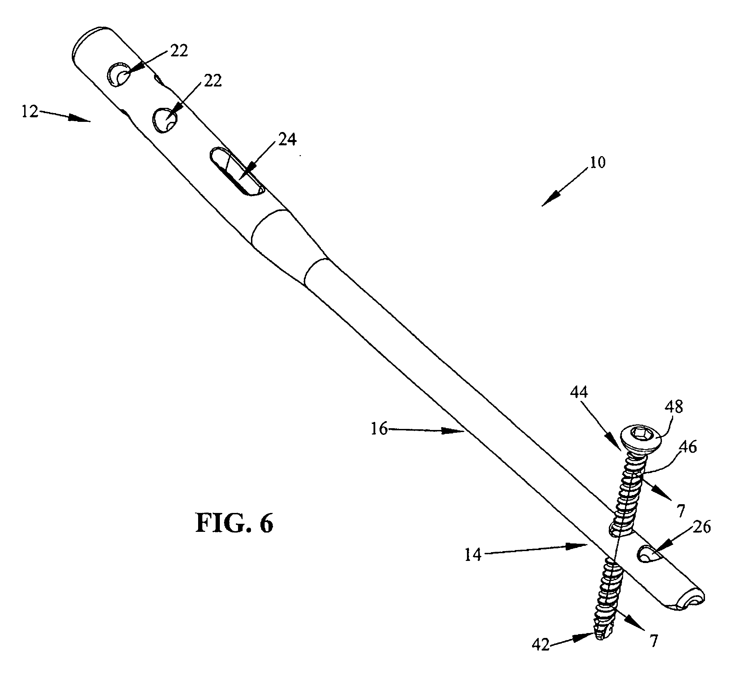

Figure 6 depicts screw 40 inserted into a bore 26, as would be accomplished in numerous surgical techniques in which nail 10 has been inserted into the canal (not shown) of a bone (not shown). A bone has been omitted from Figure 6 for the sake of clarity. In addition, it should be noted that nail 10 may be configured to be located within the canal of numeral bones, in accordance with numeral surgical techniques. Once nail 10 has been properly positioned within a bone, screw 40 may be inserted into both the bone and the nail 10 in order to retain the nail in a fixed position with respect to the bone.

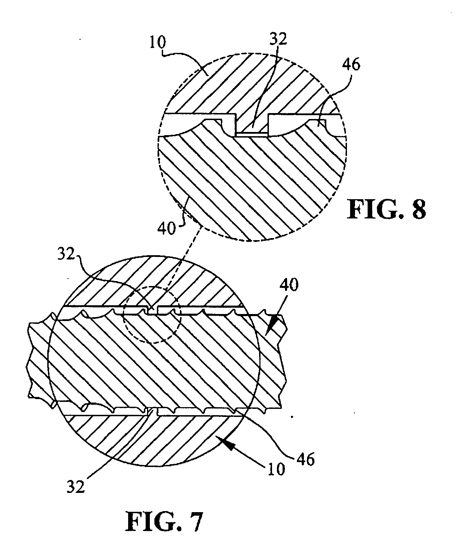

Figures 7 and 8 are section views taken along the section line 7-7 of Figure 6 depicting screw 40 inserted into bore 26 of nail 10. As depicted in the Figures, the threads 46 engage engagement portions 32 of nail 10 by receipt of engagement portions 32 between adjacent crests of thread 46 of screw 42 as the same is threaded through bore 26 of nail 10. The engagement between the engagement portions 32 and screw 40 is sufficient to ensure that the screw 40 and nail 10 remain in a relatively fixed position with respect to each other. It should be noted that without engagement portions 32, the screw 40 would be able to freely traverse within bore 26. Engagement portions 32, however, require that the screw 40 be rotated in a traditional fashion whenever being inserted into or removed from bore 26. Accordingly, engagement portions 32 result in a more robust engagement between screw 40 and nail 10 than would occur if engagement portions 32 were not present in nail 10. It should be noted that in embodiments, the engagement portions may be sized and configured to allow the threads 40 of the screw 42 to deform, or tap, the engagement portions 32 during a first insertion of screw 42.

In the depicted embodiment, the substantially smooth portions 28, 30 may provide a surgeon with a guide when screw 40 is first being inserted into bore 26. When screw 40 has traversed a sufficient distance of bore 26 with one of the smooth portions 28, 30 guiding screw 40, thread 42 may then engage engagement portions 32.

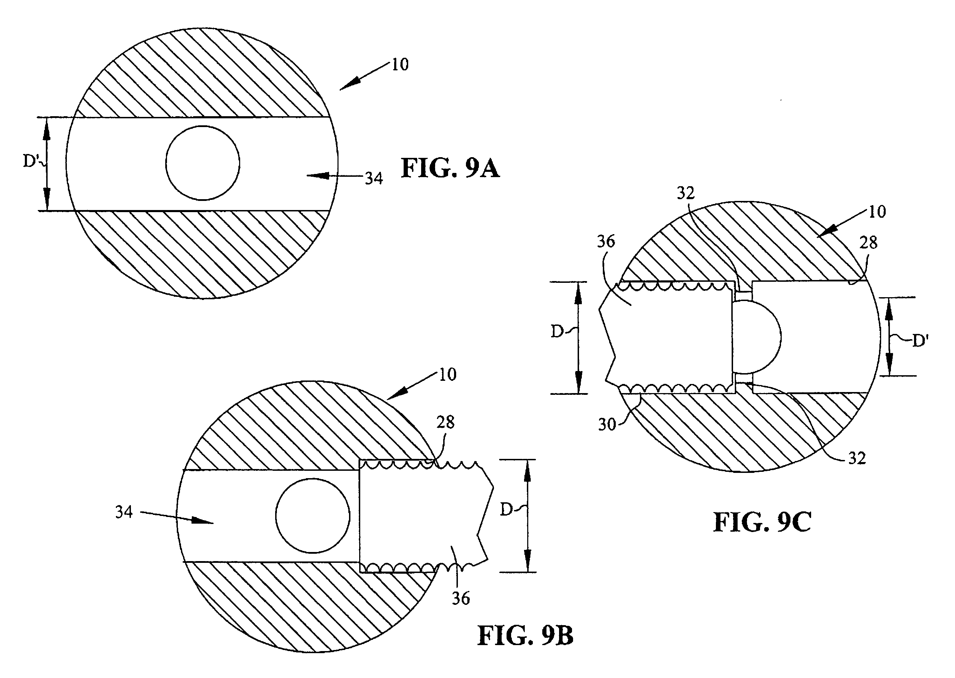

Engagement portions 32 may be formed in any suitable manner, such as by the process depicted in Figures 9A - 9C. Figure 9A depicts a section view of nail 10 including unformed through bore 34. Unformed through bore 34 has a substantially uniform diameter approximately equal to diameter D'. A workpiece capable of removing material, such as a mill 36, depicted in Figure 9B, may be inserted into unformed bore 34 in order to remove material from the bore 34. As mill 36 removes material, mill 36 forms smooth portion 28 having a diameter D. Workpiece 36 may then inserted into the opposite end of bore 34 in order to form smooth second portion 30, as shown in Figure 9C. As set forth above, smooth second portion 30 has a diameter substantially equal to diameter D. In forming smooth second portion 30, the maximum distance workpiece 36 is inserted should not exceed a distance that allows workpiece 36 to reach smooth first portion 28. By not reaching smooth first portion 28, workpiece 36 forms the engagement portions 32. As shown in Figure 9C, since workpiece 36 has a substantially flat bottom portion 38, the engagement portions 36 have a substantially rectangular cross section. It should be noted that in embodiments, diameter D may be approximately equal to the outer diameter of screw 40 wherein the thread 42 of screw 40 would deform, or tap, the engagement portions 32 during insertion of screw 40.

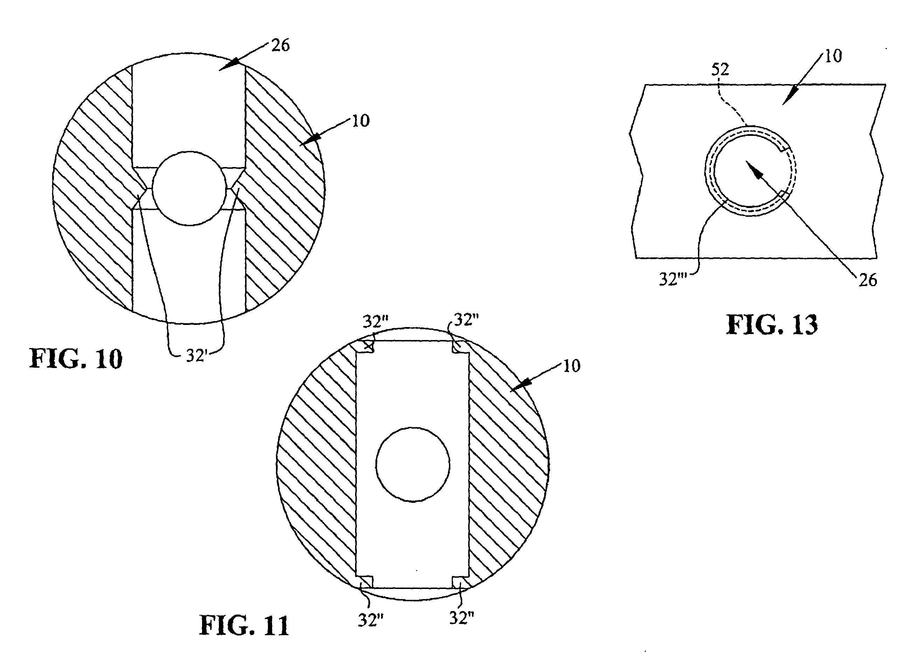

Figure 10 depicts a cross section of another embodiment of the through bore 22. In the depicted embodiment, the engagement portions 32' are formed with a workpiece such as a countersink, which has an angled end. Accordingly, the cross section of the engagement portions 32' are triangular when formed in the manner depicted in Figure 9A - 9C and described above but utilizing a countersink rather than a mill 36.

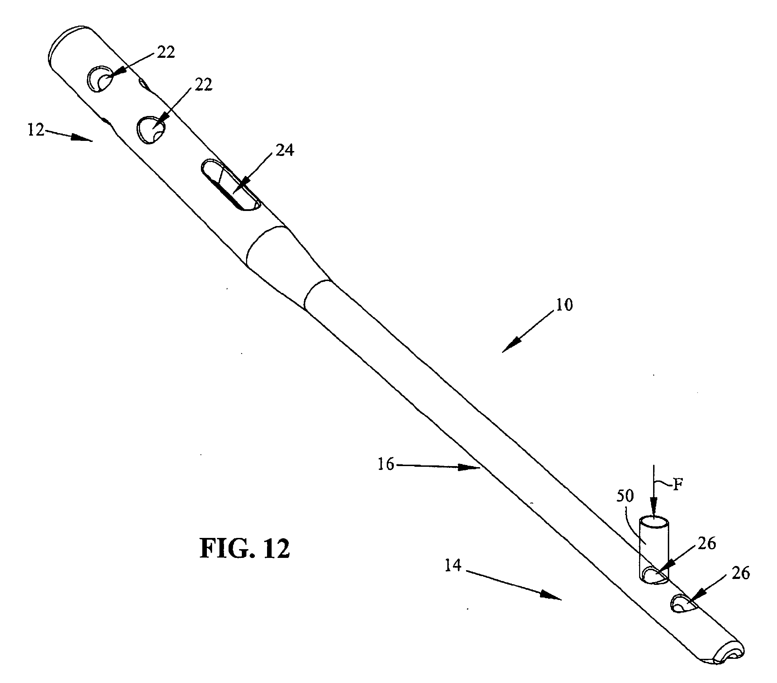

Figure 11 depicts a cross section of another embodiment of bore 26. In the embodiment depicted in Figure 11, the engagement portions 32" are formed by swaging the bore 26. For example, Figure 12 depicts a method of swaging bore 26. As shown in Figure 12, a bar 50 may be placed across the through bore 26. Bar 50 may then be struck with an object capable of providing a force in the direction of arrow F, such as a hammer or mallet, for example. The force imparted upon the bore 26 by the bar 50 causes a portion of the bore 26 to deform, thereby forming engagement portions 32". Swaging may also be used to create engagement portions 32" on the opposite side of bore 26, as depicted in Figure 11, in a similar manner. In addition, other known mechanisms capable of swaging bore 26 may be utilized. Furthermore, if suitable, the bar 50 may be orientated in a variety of different positions than that depicted in Figure 12, if desired.

Figure 13 depicts a side view of another embodiment of bore 26. In the depicted embodiment, bore 26 includes at least one groove 52, illustrated in phantom, formed therein in a suitable manner. For example, a key mill (not shown) may be utilized to form a groove 52 in a known manner.

A c-ring 32''' may then be located in each of the grooves 52 in a known manner. For example, the c-ring 32''' may be compressed slightly thereby allowing the c-ring 32''' to be moved to a groove 52. Once c-ring 32''' is located in groove 52, c-ring 32''' may be released thereby allowing c-ring 32''' to expand and reside within groove 52. If desired, an adhesive may be utilized to help retain the c-ring 32''' within groove 52. C-ring 32''' may be formed from any suitable material, such as a plastic.

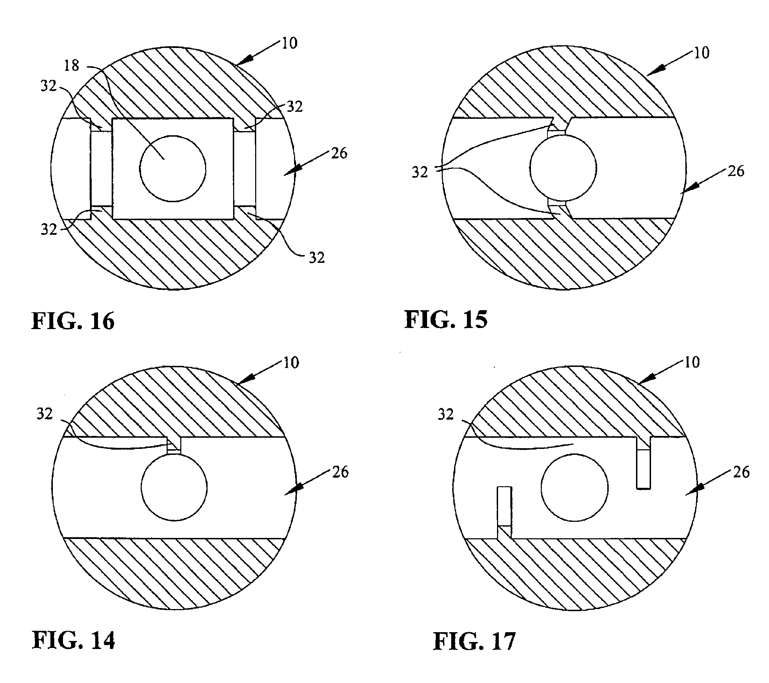

Figures 14 through 17 depict additional modifications that may be made to the engagement portions 32 depicted in Figure 4. For example, in Figure 14, one of the engagement portions 32 has been removed, thereby leaving a single engagement portion 32. The removed engagement portion may be removed in any suitable fashion, such as with a mill, for example.

In Figure 15, the engagement portions 32 are askew, i.e. projected at an angle with respect to the longitudinal axis of bore 26. The configuration depicted in Figure 15 may be formed in any suitable manner, such as by impacting the engagement portions 32 with a forming device or machining the engagement portions with a countersink from one direction and a mill in the opposite direction.

Figure 16 depicts an embodiment including multiple engagement portions formed in bore 26 on opposite sides of cannula 18.

Figure 17 depicts an embodiment wherein each engagement portion 32 is formed on opposite sides of cannula 18 and the engagement portions are axially spaced with respect to one another along the longitudinal axis of bore 26. It should be noted that each of these configurations may be utilized in placing the engagement portions 32', 32", 32'" depicted in previous figures.

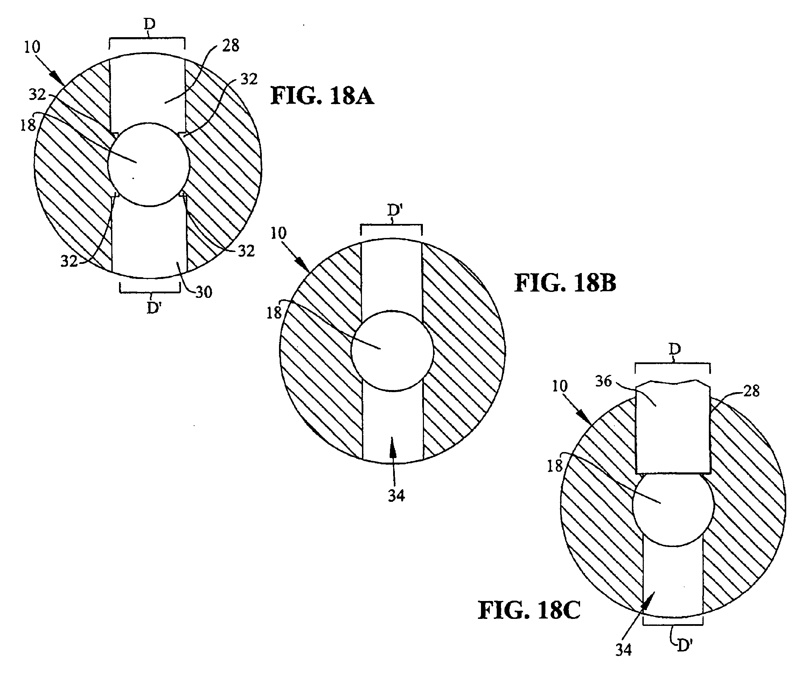

Figure 18A depicts a section view of an embodiment wherein the engagement portions 32 are formed proximate cannula 18. The embodiment depicted in Figure 18A may be formed from any suitable manufacturing process, including the process depicted in Figures 18B and 18C. As shown in Figure 18B, nail 10 includes an unformed bore 34 having a diameter of D'. As shown in Figure 18C, a workpiece capable of removing material, such as a mill 36, may be inserted into unformed bore 34 in order to remove material from the bore 34. As mill 36 removes material, mill 36 forms smooth portion 28 having a diameter D, which is larger than diameter D'. Workpiece 36 may then be inserted into the opposite end of unformed bore 34 in order to form smooth portion 30, as shown in Figure 18A. The resulting embodiment includes engagement portions 32 located proximate cannula 18.

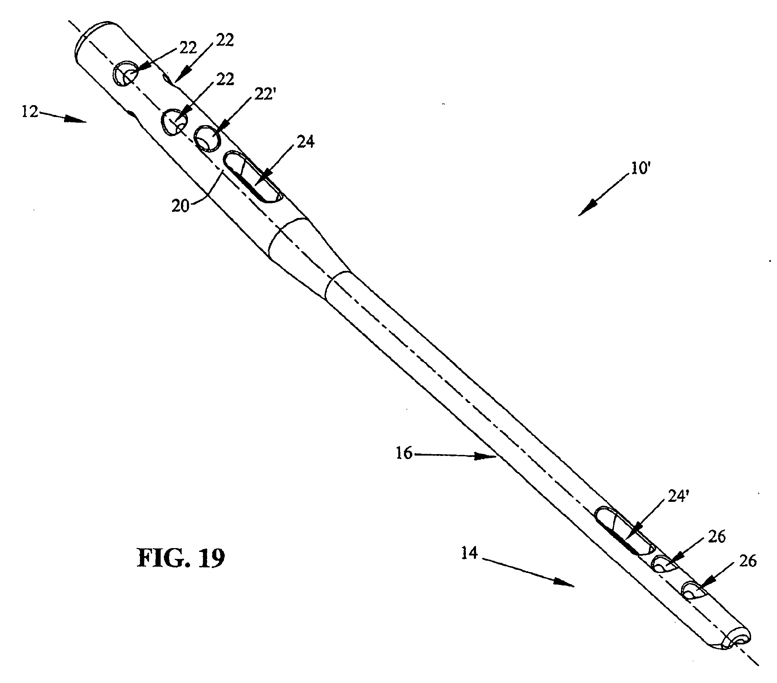

As should be understood by one with skill in the art, bores 22, 24 may also include engagement portions representative of any embodiment previously described. It should also be noted that in embodiments, the bores 22, 24, 26 may be orientated an oblique angle with respect to longitudinal axis 20. For example, as depicted an embodiment of nail 10' illustrated in Figure 19, bore 22' is an example of a bore located at an oblique angle with respect to the longitudinal axis 20 of nail 10'.

Furthermore, as is also depicted in Figure 19, an additional elongated bore 24' similar to elongated bore 24 may also be located proximate distal end 14 in embodiments. In addition, it should be noted that in any elongated bores 24, 24', the engagement portions will allow for dynamization of the nail 10' when desired. Moreover, the elongated bores 24, 24' also allow for the insertion of a bone screw at an oblique angle with respect to the longitudinal axis 20 of nail 10'.

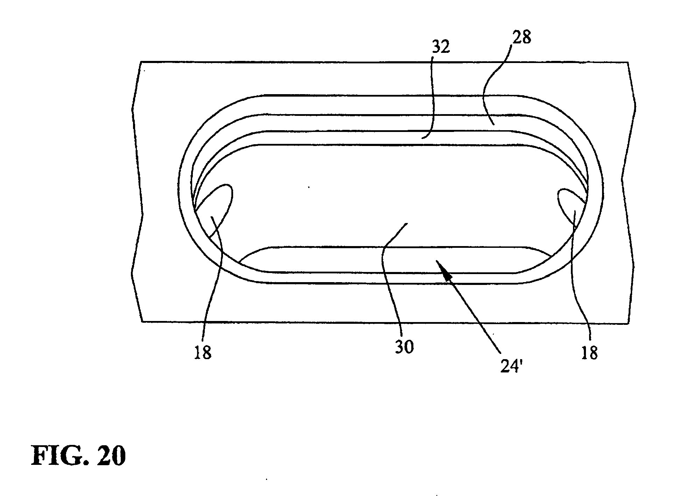

Figure 20 depicts an embodiment of elongated bore 24' including an engagement portion 32. As explained above, bore 24' may include suitable engagement portion including those described previously. As illustrated in Figure 20, in the depicted embodiment, bore 24' includes smooth portions 28, 30 positioned with the engagement portion 32 located intermediate. Furthermore, as illustrated in Figure 20, engagement portion 32 does not align with cannula 18. In embodiments, engagement portion 32 may align with cannula 18 thereby ensuring the smooth portions 28, 30 are of approximately equivalent size. It should be noted that in all embodiments of the bores 22, 24, 26, the engagement portion 32 may be located at any suitable position therein.

While this invention has been described as having exemplary designs, the present invention may be further modified within the spirit and scope of the disclosure. This application is therefore intended to cover any variations, uses, or adaptations of the invention using its general principles. Further, this application is intended to cover such departures from the present disclosure as come within known or customary practice in the art to which this invention pertains.

## Claims

1. An intramedullary nail configured to receive at least a portion of a bone screw, said nail comprising:
a body portion having first and second ends; and
at least one bore in said body portion, said bore configured to receive the bone screw, wherein at least a portion of said bore is substantially smooth and said bore comprises at least one member configured to engage at least a portion of a thread of the screw.

2. The intramedullary nail of claim 1 **characterised in that** said second end includes at least one bore configured to receive a second screw, said bore including at least one member configured to engage at least a portion of a thread of said second screw.

3. The intramedullary nail of claim 1 **characterised in that** said first end comprises two bores, each said bore configured to receive a screw and comprising a member configured to engage at least a portion of the thread of the screw.

4. The intramedullary nail of claim 1 charterised in that said member includes at least a portion having a substantially rectangular cross section.

5. The intramedullary nail of claim 1 **characterised in that** said member includes at least a portion having a substantially triangular cross section.

6. The intramedullary nail of claim 1 **characterised in that** it further includes a cannula extending proximate at least a portion of a longitudinal axis of said nail; wherein said bore comprises at least two members and said cannula is at least partially located intermediate said members.

7. The intramedullary nail of claim 1 **characterised in that** said member comprises a c-ring.

8. The intramedullary nail of claim 7 **characterised in that** said bore further comprises a recessed area configured to receive said c-ring.

9. The intramedullary nail of claim 1 **characterised in that** said member is located proximate an end of said bore.

10. The intramedullary nail of claim 1 **characterised in that** said member is integrally formed within said bore.

11. An intramedullary nail configured to receive at least a portion of the bone screw **characterised in that**, said nail comprises:
a first end comprising at least one bore configured to receive the screw;
a second end;
a body portion intermediate said first end and said second end;
wherein said at least one bore comprises a substantially smooth portion and means for restraining transverse movement of the screw within said bore.

12. The intramedullary nail of claim 11 **characterised in that** said second end comprises at least one bore comprising means for restraining transverse movement of the screw within said bore.

13. The intramedullary nail of claim 11 **characterised in that** said bore includes a second means for restraining transverse movement of the screw within said bore.

14. The intramedullary nail of claim 13 **characterised in that** it further comprises a shaft extending substantially parallel to a longitudinal axis of said nail, wherein at least a portion of said shaft is positioned intermediate said first means and said second means.

15. An implant system configured to affix an intramedullary nail to a bone following insertion into a canal of the bone; **characterised in that** the system comprises:
a bone screw having at least one thread;
a bore comprising at least one substantially smooth portion and at least one member configured to engage at least a portion of said thread of said screw.

16. The implant system of claim 15 **characterised in that** said bore is positioned within an end of the nail.

17. The implant system of claim 15 **characterised in that** said means for restraining comprises a member extending into said bore from a surface defining said bore.

18. The implant system of claim 17 **characterised in that** said member is integrally formed in said surface.

19. The implant system of claim 15 **characterised in that** said bore further comprises a second engagement member.

20. The implant system of claim 15 **characterised in that** said bore further includes a second substantially smooth portion, said engagement member positioned intermediate said substantially smooth portions.

21. An implant system configured to affix an intramedullary nail to a bone following insertion into a canal of the bone; **characterised in that** the system comprises:
a bone screw having at least one thread;
a bore comprising at least one substantially smooth portion and means for restraining transverse movement of the screw within said bore.
